# EUROPÄISCHE PATENTSCHRIFT

(11) **EP 1 277 488 B1**
(45) Veröffentlichungstag und Bekanntmachung des Hinweises auf die Patenterteilung: **15.09.2004**
(21) Anmeldenummer: 02012751.0
(22) Anmeldetag: 08.06.2002
(51) Int. Cl.: A61M 16/10

(54) **Vorrichtung zur Filterung von Atemluft**
Filtering device for respiratory air
Dispositif de filtrage de l'air respiré

(30) Priorität: 17.07.2001 DE 10134725
(43) Veröffentlichungstag der Anmeldung: 22.01.2003
(73) Patentinhaber: Weinmann Geräte für Medizin GmbH & Co. KG, 22525 Hamburg (DE)
(72) Erfinder: Feldhahn, Karl-Andreas, Dr., 22761 Hamburg (DE); Peasch, Rainer, 25451 Quickborn (DE)
(74) Vertreter: Klickow, Hans-Henning, Dr.-Ing.

(56) Entgegenhaltungen:
- EP-A- 1 023 912
- US-A- 4 123 091
- US-A- 5 778 872

## Beschreibung

Die Erfindung betrifft eine Vorrichtung zur Filterung von Atemluft, die ein Filtergehäuse aufweist, das aus einem Gehäuseunterteil und einem Gehäuseoberteil ausgebildet ist und bei der vom Filtergehäuse ein Filterelement gehaltert ist sowie bei der sich durch das Filtergehäuse hindurch eine Leitung zur Druckübertragung erstreckt, die im Bereich von Endsegmenten jeweils mit einer Kupplung zur Verbindung mit einer Druckmeßleitung ausgestattet ist

Derartige Vorrichtungen werden in der Regel im Bereich einer Atemleitung zwischen einem Beatmungsgerät und einer Atemmaske eines Patienten eingesetzt, um aus einer Umgebung angesaugte Verunreinigungen zurückzuhalten. Ein Problem bei der Verwendung derartiger Filter besteht darin, daß insbesondere bei Beatmungsgeräten zur Durchführung der CPAP-Therapie von der Beatmungsmaske zum Beatmungsgerät eine Druckmeßleitung verläuft, die den Filterbereich zur Gewährleistung einer ausreichenden Meßgenauigkeit überbrücken muß. Die Inbetriebnahme sowie die Auswechselung der Filterelemente konnten aufgrund dieser Notwendigkeit zur Verlegung der Druckmeßleitung bislang noch nicht alle Anforderungen erfüllen, die an eine einfache Handhabung gestellt werden.

Aus der EP 1 023 912 ist es bereits bekannt, eine Vorrichtung zur Filterung von Atemluft im Bereich eines Atemgasschlauches anzuordnen, der ein Beatmungsgerät mit einer Atemmaske verbindet. Das Filtergehäuse besteht aus einem Gehäuseunterteil und einem Gehäuseoberteil. Innerhalb des Filtergehäuses ist ein Filterelement gehaltert, durch das sich ein Druckmeßschlauch hindurcherstreckt. Der Druckmeßschlauch weist im Bereich seines dem Beatmungsgerät zugewandten Endsegmentes eine Kupplung auf. Im Bereich seines der Atemmaske zugewandten Endes ragt der Druckmeßschlauch mit einem Endsegment in ein Anschlußelement der Atemmaske hinein.

Aus der US 5,778,872 ist ebenfalls eine Vorrichtung zur Filterung von Atemluft bekannt, die ein Filtergehäuse aufweist, das aus einem Gehäuseunterteil und einem Gehäuseoberteil besteht. Innerhalb des Filtergehäuses ist ein Filterelement gehaltert. Durch den Hauptfilter erstreckt sich ein Innengehäuse hindurch, das ebenfalls mit einem separaten Filterelement versehen ist. Es wird hierdurch ein koaxialer Filterbereich bereitgestellt, der eine voneinander getrennte Filterung der inspiratorischen sowie der expiratorischen Gasströmung ermöglicht. Aufgrund des im Bereich des Innengehäuses angeordneten separaten Filterelementes ist das Innengehäuse nicht für eine Druckübertragung geeignet, da das innere Filterelement zu Druckverlusten führt und darüber hinaus zur Unterstützung der Beatmung eine ausreichende volumenströmung unterstützt werden muß.

In der US 4,123,091 wird zur Verbindung von Segmenten einer pneumatischen Leitung ein Klick-Verschluß beschrieben, der ein einfaches Einrasten sowie voneinander Trennen der Kupplungsteile unterstützt.

Aufgabe der vorliegenden Erfindung ist es, eine Vorrichtung der einleitend genannten Art derart zu konstruieren, daß die Handhabung erleichtert und Druckverluste vermieden werden.

Diese Aufgabe wird erfindungsgemäß dadurch gelöst, daß eines der Endsegmente mit einem Dichtelement versehen ist und daß die Endsegmente der Leitung zur Druckübertragung in die Kupplungsstutzen hineinragen.

Die Verlegung der Druckmeßleitung durch das Filtergehäuse hindurch vermeidet die Handhabung von separaten außenseitigen Schläuchen. Durch die Ausstattung der Leitung mit den Kupplungen ist es darüber hinaus möglich, ein modulares System bereitzustellen, bei dem die Vorrichtung in einfacher Weise an entsprechende Gegenkupplungen einer Atemleitung oder des Beatmungsgerätes angeschlossen werden kann.

Bei entsprechend aufeinander abgestimmten Kupplungselementen ist es insbesondere möglich, die Vorrichtung bedarfsabhängig einzusetzen und beispielsweise in einfacher Weise die Atemleitung vom Beatmungsgerät zu lösen und die Vorrichtung zwischen dem Beatmungsgerät und der Atemleitung einzusetzen. Die Verwendung der Kupplungen ermöglicht es, die erläuterten Arbeitsschritte mit äußerst kurzem Zeitaufwand durchzuführen.

Zur Erleichterung einer Herstellbarkeit wird vorgeschlagen, daß das Gehäuseoberteil mit einem Kupplungsstutzen versehen ist.

Die Bereitstellung eines modularen Systems wird dadurch unterstützt, daß der Kupplungsstutzen des Gehäuseoberteiles als ein Kupplungsaußenteil ausgebildet ist.

Ebenfalls trägt es zur einfachen Herstellbarkeit bei, daß das Gehäuseunterteil einen Kupplungsstutzen aufweist.

Ein weiterer Beitrag zur Erreichung eines modularen Systems wird dadurch geliefert, daß der Kupplungsstutzen des Gehäuseunterteiles als ein Kupplungsinnenteil ausgebildet ist.

Eine einfache Anschließbarkeit an einen Druckmeßschlauch wird dadurch bereitgestellt, daß die Endsegmente der Leitung zur Druckübertragung in die Kupplungsstutzen hineinragen.

Zur Vermeidung von Druckverlusten wird vorgeschlagen, daß eines der Endsegmente mit einem Dichtelement versehen ist.

Eine einfach Montierbarkeit wird dadurch unterstützt, daß das Dichtelement auf das Endsegment aufgesteckt ist.

Zur Unterstützung eines Toleranzausgleiches bei einem Zusammenfügen der Kupplungselemente wird vorgeschlagen, daß das Dichtelement einen Einführkonus aufweist.

Insbesondere ist daran gedacht, daß der Einführkonus trichterförmig in eine Verbindungsausnehmung übergeleitet ist.

Eine zweckmäßige Materialauswahl zur Erreichung einer Druckabdichtung besteht darin, daß das Dichtelement aus einem Elastomer ausgebildet ist.

Eine einfache Herstellbarkeit der Vorrichtung wird auch dadurch unterstützt, daß die Leitung zur Druckübertragung mindestens zweiteilig ausgebildet ist.

Zur Gewährleistung einer konstruktiv einfachen Gestaltung wird vorgeschlagen, daß ein Leitungsunterteil der Leitung mit einem Leitungsoberteil zusammensteckbar ist.

Die Bereitstellung eines Systems aus einfachen und modular zusammensteckbaren Komponenten wird dadurch unterstützt, daß die Endsegmente und die Kupplungsstutzen relativ zueinander koaxial angeordnet sind.

Zur Gewährleistung geringer Betriebskosten wird vorgeschlagen, daß das Filterelement auswechselbar im Filtergehäuse gehaltert ist.

Ein an unterschiedliche Anwendungsanforderungen anpaßbares Gesamtsystem wird dadurch bereitgestellt, daß das Filtergehäuse modular mit der Atemleitung zusammensteckbar ist.

Eine besonders einfache Handhabung kann dadurch erreicht werden, daß die Kupplungselemente des Filtergehäuses und der Atemleitung Klick-Verschlüsse ausbilden.

Eine einfache Gestaltung bei zugleich einfacher Bedienung und langer Betriebsfähigkeit kann dadurch erreicht werden, daß das Filtergehäuse eine Arretierung aufweist, die als ein federnd angeordneter Schwenkhebel ausgebildet ist.

In der Zeichnung sind Ausführungsbeispiele der Erfindung schematisch dargestellt. Es zeigen:
- Fig. 1: eine perspektivische Darstellung eines Beatmungsgerätes mit angeschlossener Atemmaske sowie Atemleitung,
- Fig. 2: einen Längsschnitt durch ein Filtergehäuse,
- Fig. 3: eine Seitenansicht eines Gehäuseunterteiles des Filtergehäuses,
- Fig. 4: einen Längsschnitt gemäß Schnittlinie IV-IV in Fig. 3,
- Fig. 5: eine Seitenansicht gemäß Blickrichtung V in Fig. 3,
- Fig. 6: eine teilweise Darstellung einer Seitenansicht gemäß Blickrichtung VI in Fig. 4,
- Fig. 7: eine Seitenansicht eines Gehäuseoberteiles des Filtergehäuses,
- Fig. 8: eine teilweise Darstellung eines Längsschnittes gemäß Schnittlinie VIII-VIII in Fig. 7,
- Fig. 9: einen Querschnitt gemäß Schnittlinie IX-IX in Fig. 7,
- Fig. 10: eine Seitenansicht gemäß Blickrichtung X in Fig. 7,
- Fig. 11: einen Längsschnitt gemäß Schnittlinie XI-XI in Fig. 10,
- Fig. 12: eine teilweise Darstellung einer Atemleitung und
- Fig. 13: eine teilweise Darstellung eines Längsschnittes gemäß Schnittlinie XIII-XIII in Fig. 12.

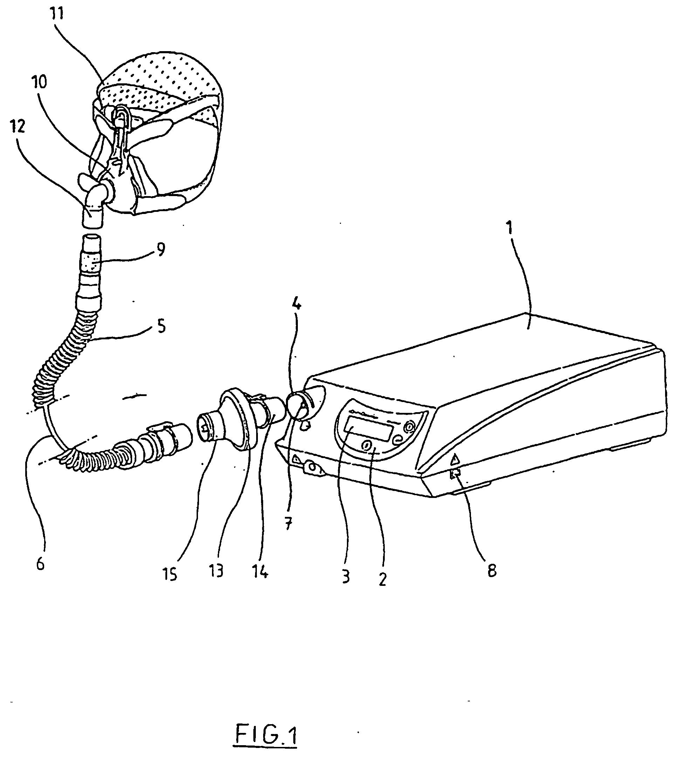

Fig. 1 zeigt den grundsätzlichen Aufbau einer Vorrichtung zur Beatmung. Im Bereich eines Gerätegehäuses (1) mit Bedienfeld (2) sowie Anzeige (3) ist in einem Geräteinnenraum ein Gebläse angeordnet. Über einen Kopplungsstutzen (4) des Gerätegehäuses (1) wird eine Atemleitung (5) angeschlossen. Entlang der Atemleitung (5) kann ein zusätzlicher Druckmeßschlauch (6) verlaufen, der über einen Druckeingangsstutzen (7) mit dem Gerätegehäuse (1) verbindbar ist. Zur Ermöglichung einer Datenübertragung weist das Gerätegehäuse (1) eine Schnittstelle (8) auf.

Im Bereich einer dem Gerätegehäuse (1) abgewandten Ausdehnung der Atemleitung (5) ist ein Ausatmungselement (9) angeordnet.

Fig. 1 zeigt darüber hinaus eine Beatmungsmaske (10), die als Nasalmaske ausgebildet ist. Eine Fixierung im Bereich eines Kopfes eines Patienten kann über eine Kopfhaube (11) erfolgen. Im Bereich ihrer der Atemleitung (5) zugewandten Ausdehnung weist die Beatmungsmaske (10) einen Anschlußstutzen (12) auf.

Zwischen der Atemleitung (5) und das Gerätegehäuse (1) ist ein Filtergehäuse (13) einsetzbar, das über Kupplungsstutzen (14, 15) mit der Atemleitung (5) einerseits und mit dem Kopplungsstutzen (4) des Gerätegehäuses (1) anderseits verbindbar ist.

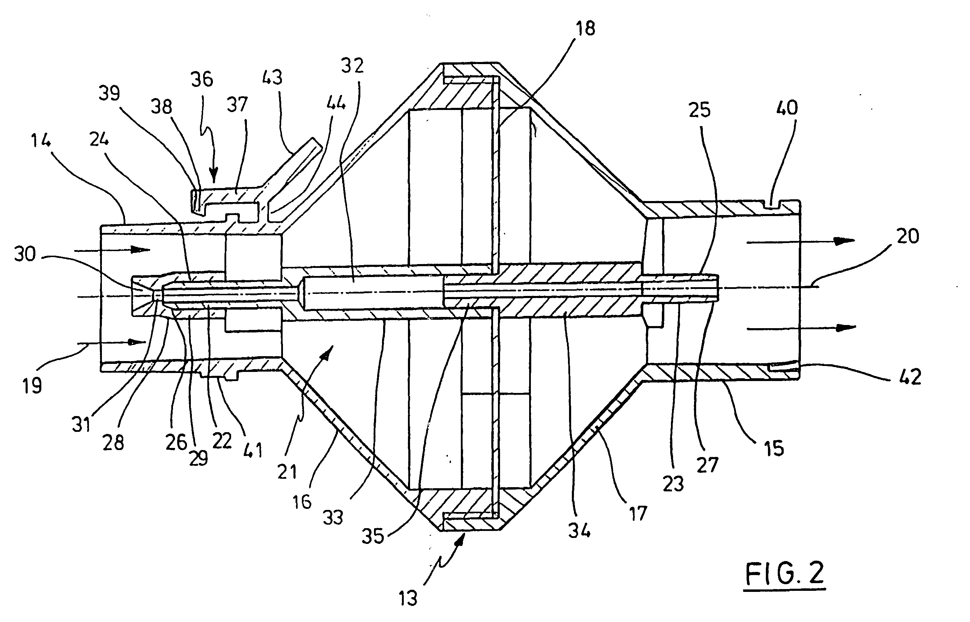

Aus dem Längsschnitt in Fig. 2 ist erkennbar, daß das Filtergehäuse (13) aus einem Gehäuseunterteil (16) und einem Gehäuseoberteil (17) ausgebildet ist. Vom Gehäuseunterteil (16) und vom Gehäuseoberteil (17) wird gemeinsam ein Filterelement (18) gehaltert. Ausgehend vom Filterelement (18) verjüngt sich das Gehäuseunterteil (16) entgegen einer vorgesehenen Strömungsrichtung (19) mit einer Orientierung auf den Kupplungsstutzen (14), der zur Verbindung mit dem Gerätegehäuse (1) des Beatmungsgerätes dient. Das Gehäuseoberteil (17) verjüngt sich entlang einer Filterlängsachse (20) in Richtung auf den Kupplungsstutzen (15), der zum Anschluß an die Atemleitung (5) vorgesehen ist.

Durch das Filtergehäuse (13) hindurch erstreckt sich eine Leitung (21) zur Druckübertragung, die im Bereich von Endsegmenten (22, 23) jeweils mit einer Kupplung (24, 25) zur Verbindung mit der in Fig. 2 nicht dargestellten Atemleitung (5) ausgestaltet ist.

Die Kupplungen (24, 25) sind jeweils als Stutzen ausgebildet, die in ihren dem Filterelement (18) abgewandten Endbereichen mit außenseitigen Verjüngungen (26, 27) versehen sind. Auf die Endsegmente (22, 23) sind Dichtelemente (28) aufsteckbar, die vorzugsweise aus einem elastomeren Material ausgebildet sind. Vorzugsweise wird lediglich auf eines der Endsegmente (22, 23) ein Dichtelement (28) aufgesteckt, so daß eine modulare Zusammensteckbarkeit einer Mehrzahl von Bauelementen unterstützt wird.

Das Dichtelement (28) erstreckt sich mit einer Elementbasis (29) im wesentlichen hülsenförmig entlang des zugeordneten Endsegmentes (22, 23). Im Bereich seiner dem Endsegment (22, 23) abgewandten Ausdehnung ist das Dichtelement (28) mit einem Einführkonus (30) versehen, der eine Abdichtung und Zentrierung einer zugeordneten Gegenkupplung unterstützt. Der Einführkonus (30) ist über eine Verbindungsausnehmung (31) trichterförmig in einen Innenraum (32) der Leitung (21) zur Druckübertragung übergeleitet.

Die Leitung (21) zur Druckübertragung ist in ein Leitungsunterteil (33) und ein Leitungsoberteil (34) unterteilt, die sich vorzugsweise zentrisch relativ zur Filterlängsachse (20) erstrecken und in einer Umgebung des Filterelementes (18) zusammensteckbar sind. Die Zusammensteckbarkeit kann beispielsweise durch eine Verjüngung (35) des Leitungsoberteiles (34) realisiert sein, die in eine entsprechende Ausnehmung des Leitungsunterteiles (33) abgedichtet einführbar ist.

Die Halterung des Filterelementes (18) erfolgt vorzugsweise derart, daß eine Austauschbarkeit des Filterelementes (18) gegeben ist. Dies kann durch eine Befestigung des Gehäuseoberteiles (17) am Gehäuseunterteil (16) beispielsweise in Form einer Schraubverbindung oder eines Bajonettverschlusses realisiert sein. Ebenfalls ist eine Verbindung durch Rastelemente oder federnde Elemente möglich.

Zur Unterstützung einer modularen Kombinierbarkeit der einzelnen Bestandteile des Beatmungssystems ist insbesondere daran gedacht, den Kupplungsstutzen (14) mit einem Außendurchmesser zu versehen, der einem Innendurchmesser des Kupplungsstutzens (15) entspricht. Der Kupplungsstutzen (14) ist außenseitig darüber hinaus mit einer Arretierung (36) ausgestattet, die als ein verschwenkbarer Hebel (37) ausgebildet ist, der mit einer Rastung (38) versehen ist. Die Rastung (38) weist eine Einführanschrägung (39) auf, die bei einem Zusammenfügen der Bauelemente die Rastung (38) in den Bereich einer Vertiefung (40) des zugeordneten Kupplungselementes leitet. Eine korrekte Orientierung der Bauelemente in Rotationsrichtung um die Filterlängsachse (20) herum wird durch eine zugeordnete Phase (41) und eine Ausrichtvertiefung (42) am zugeordneten Kupplungselement unterstützt.

Zu einem Lösen der Kupplungsteile wird die Arretierung (36) durch einen Druck auf eine Bedienfläche (43) um einen Schwenksteg (44) herum verdreht und hierdurch die Rastung (38) aus der Vertiefung (40) herausgeführt. In diesem Zustand können die Kupplungsteile in einfacher Weise auseinander gezogen werden.

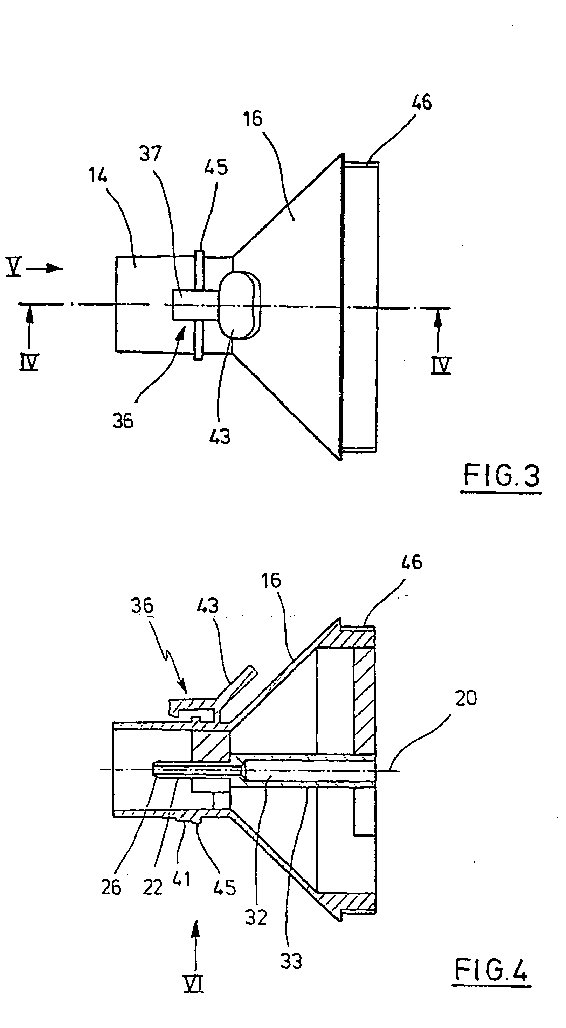

Fig. 3 zeigt eine Seitenansicht des Gehäuseunterteiles (16). Es ist insbesondere die Anordnung der Arretierung (36) relativ zu einem Anschlagsteg (45) zu erkennen, der bei einem Zusammenfügen mehrerer Kupplungsteile eine definierte Endstellung vorgibt. Ebenfalls ist erkennbar, daß das Gehäuseunterteil (16) im Bereich seiner dem Gehäuseoberteil (17) zuwendbaren Ausdehnung eine Verjüngung (46) aufweist, die ein Zusammenfügen mit dem Gehäuseoberteil (17) unterstützt.

Fig. 4 zeigt das Gehäuseunterteil (16) ähnlich zur Darstellung in Fig. 2, jedoch ohne ein Zusammenfügen mit dem Gehäuseoberteil (17) sowie ohne aufgestecktes Dichtelement (28).

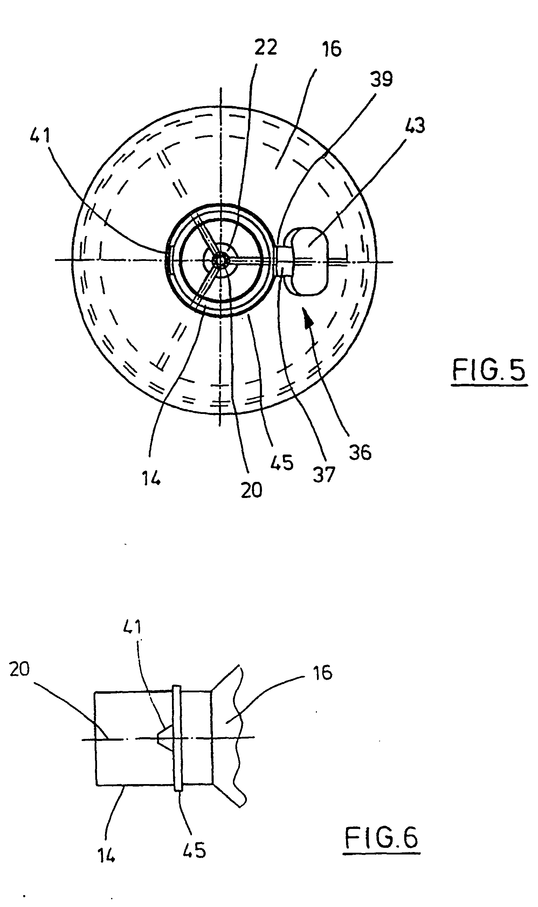

Fig. 5 veranschaulicht die im wesentlichen koaxiale Anordnung relativ zur Filterlängsachse (20) des Gehäuseunterteiles (16), des Kupplungsstutzens (14) sowie des Endsegmentes (22).

Fig. 6 veranschaulicht, daß sich die Phase (41) in Richtung der Filterlängsachse (20) verjüngt, um bei einem Zusammenfügen der Kupplungsteile eine Ausrichtung relativ zueinander in Umfangsrichtung bezüglich der Filterlängsachse (20) zu unterstützen.

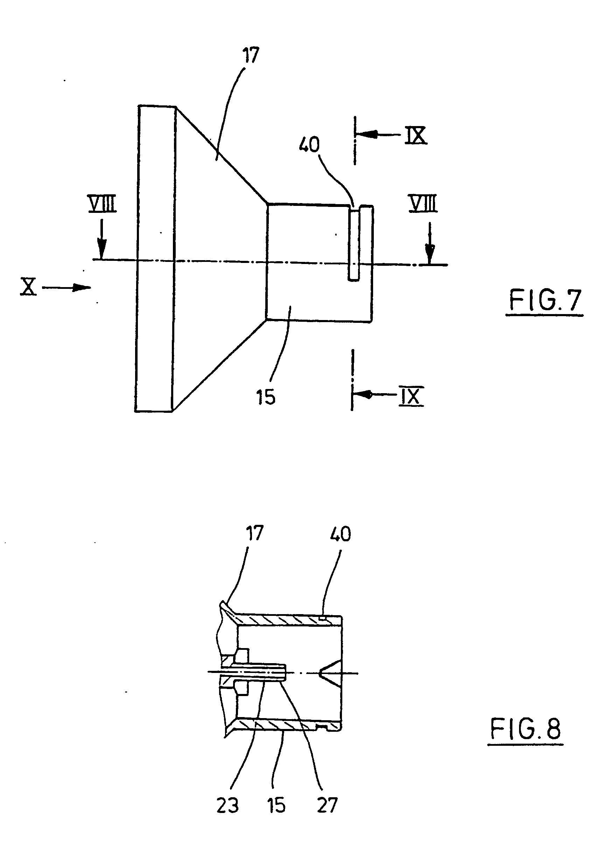

Aus der Seitenansicht des Gehäuseoberteiles (17) in Fig. 7 ist insbesondere zu erkennen, daß sich die Vertiefung (40) als Segment einer Umfangsnut im Bereich der Außenseite des Kupplungsstutzens (15) erstreckt.

Fig. 8 veranschaulicht nochmals den Übergang des Kupplungsstutzens (15) in den sich erweiternden Bereich des Gehäuseoberteiles (17) sowie die relativ zum Kupplungsstutzen (15) koaxiale Anordnung des Endsegmentes (23).

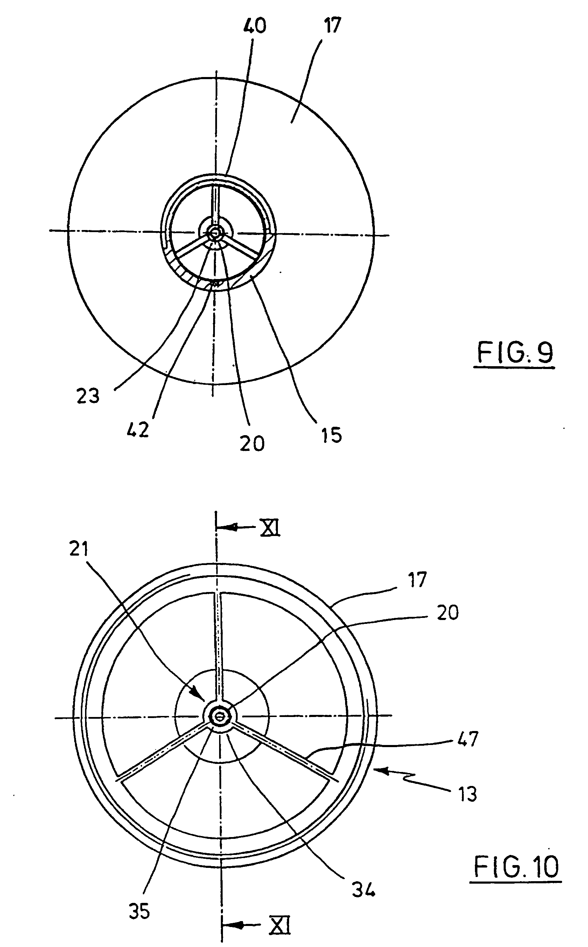

Aus Fig. 9 ist zu erkennen, daß auch im Bereich des Gehäuseoberteiles (17) eine im wesentlichen koaxiale Anordnung relativ zur Filterlängsachse (20) des Endsegmentes (23) und des Kupplungsstutzens (15) vorliegt. Ebenfalls ist der Verlauf der Vertiefung (40) als Umfangsnutsegment entlang des Umfanges des Kupplungsstutzens (15) erkennbar.

Aus Fig. 10 ist ersichtlich, daß innerhalb des Filtergehäuses (13) eine Halterung der Leitung (21) und insbesondere des Leitungsoberteiles (24) innerhalb des Gehäuseoberteiles (17) durch Radialstege (47) erfolgt, die sich ausgehend von der Leitung (21) in Richtung auf die Wandung des Filtergehäuses (13) erstrecken. Die Radialstege (47) unterstützen eine definierte Positionierung der Leitung (21) und vermeiden Positionsveränderungen bei einer wiederholten Durchführung von Kupplungsvorgängen.

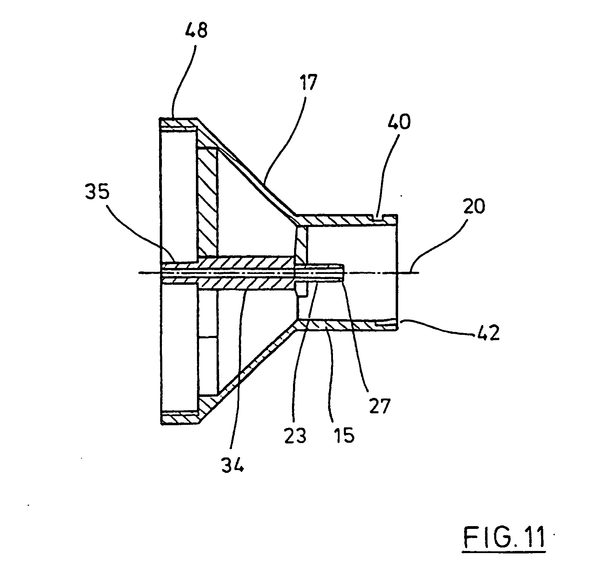

Fig. 11 zeigt das Gehäuseoberteil (17) entsprechend der Darstellung in Fig. 2, jedoch ohne ein Zusammenfügen mit dem Gehäuseunterteil (16). Zu erkennen ist insbesondere ein Einfassungssteg (48), der zur Umschließung der Verjüngung (46) des Gehäuseunterteiles (16) beim Zusammenfügen der Bauelemente dient.

Fig. 12 zeigt eine teilweise Darstellung der Atemleitung (5). Die Atemleitung (5) ist im Bereich ihrer Enden mit Schlauchkupplungen (49, 50) ausgestattet, die im wesentlichen identisch zu den Kupplungen des Filtergehäuses (13) konstruiert sind und hierdurch in einfacher weise ein Zusammenkoppeln mit den jeweiligen Gegenstücken des Filtergehäuses (13) unterstützen.

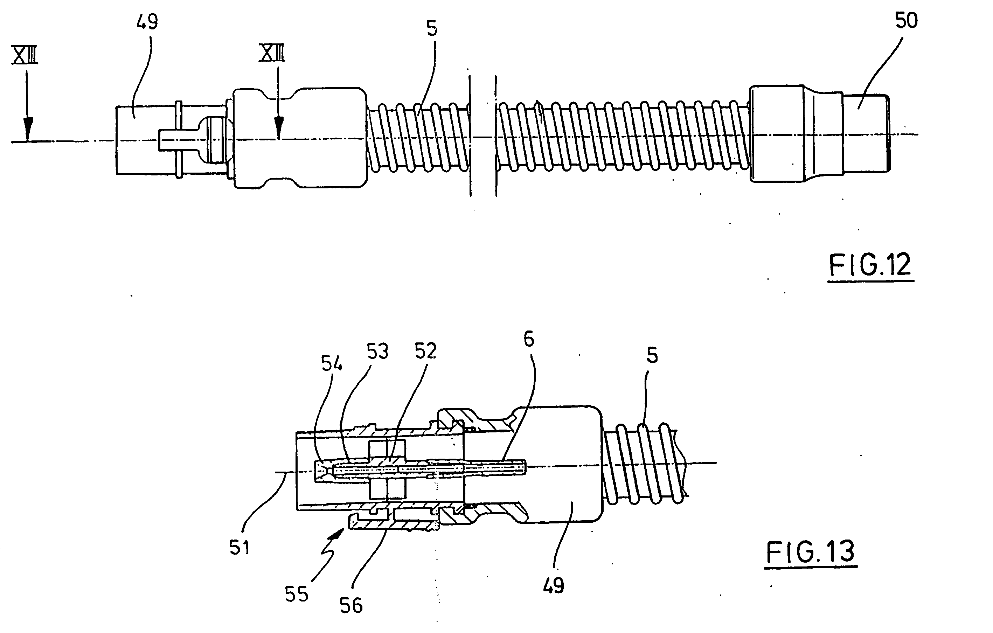

Fig. 13 zeigt einen Längsschnitt durch die Schlauchkupplung (49), deren Aufbau sehr ähnlich zu der in Fig. 2 dargestellten Kupplung realisiert ist. Durch die Atemleitung (5) erstreckt sich der Druckmeßschlauch (6) hindurch und ist mit einer im Bereich der Schlauchkupplung (49) relativ zu einer Schlauchlängsachse (51) koaxial angeordneten Druckkupplung (52) verbunden. Im wesentlichen identisch wie bei der Leitung (21) zur Druckübertragung im Bereich des Filtergehäuses (13) weist die Druckkupplung (52) ein Endsegment (53) auf, auf das ein Dichtelement (54) aufgesteckt ist, das im wesentlichen identisch zum Dichtelement (28) konstruiert ist. Ähnlich wie die Konstruktion der Rastung (38) weist auch die Schlauchkupplung (49) eine Rastung (55) auf, die im Bereich eines verschwenkbaren Hebels (56) angeordnet ist.

## Patentansprüche

1. Vorrichtung zur Filterung von Atemluft, die ein Filtergehäuse (13) aufweist, das aus einem Gehäuseunterteil (16) und einem Gehäuseoberteil (17) ausgebildet ist und bei der vom Filtergehäuse (13) ein Filterelement (18) gehaltert ist sowie bei der sich durch das Filtergehäuse (13) hindurch eine Leitung (21) zur Druckübertragung erstreckt, die im Bereich von Endsegmenten (22, 23) jeweils mit einer Kupplung (24, 25) zur Verbindung mit einer Druckmeßleitung (6) ausgestattet ist, **dadurch gekennzeichnet, daß** eines der Endsegmente (22, 23) mit einem Dichtelement (28) versehen ist und daß die Endsegmente (22, 23) der Leitung (21) zur Druckübertragung in die Kupplungsstutzen (14, 15) hineinragen.

2. Vorrichtung nach Anspruch 1, **dadurch gekennzeichnet, daß** das Gehäuseoberteil (17) mit einem Kupplungsstutzen (15) versehen ist.

3. Vorrichtung nach Anspruch 1 oder 2, **dadurch gekennzeichnet, daß** der Kupplungsstutzen (15) des Gehäuseoberteiles (17) als ein Kupplungsaußenteil ausgebildet ist.

4. Vorrichtung nach einem der Ansprüche 1 bis 3, **dadurch gekennzeichnet, daß** das Gehäuseunterteil (16) einen Kupplungsstutzen (14) aufweist.

5. Vorrichtung nach einem der Ansprüche 1 bis 4, **dadurch gekennzeichnet, daß** der Kupplungsstutzen (14) des Gehäuseunterteiles (16) als ein Kupplungsinnenteil ausgebildet ist.

6. Vorrichtung nach einem der Ansprüche 1 bis 5, **dadurch gekennzeichnet, daß** das Dichtelement (28) auf das Endsegment (22, 23) aufgesteckt ist.

7. Vorrichtung nach einem der Ansprüche 1 bis 6, **dadurch gekennzeichnet, daß** das Dichtelement (28) einen Einführkonus (30) aufweist.

8. Vorrichtung nach Anspruch 7, **dadurch gekennzeichnet, daß** der Einführkonus (30) trichterförmig in eine Verbindungsausnehmung (31) übergeleitet ist.

9. Vorrichtung nach einem der Ansprüche 1 bis 8, **dadurch gekennzeichnet, daß** das Dichtelement (28) aus einem Elastomer ausgebildet ist.

10. Vorrichtung nach einem der Ansprüche 1 bis 9, **dadurch gekennzeichnet, daß** die Leitung (21) zur Druckübertragung mindestens zweiteilig ausgebildet ist.

11. Vorrichtung nach einem der Ansprüche 1 bis 10, **dadurch gekennzeichnet, daß** ein Leitungsunterteil (33) der Leitung (21) mit einem Leitungsoberteil (34) zusammensteckbar ist.

12. Vorrichtung nach einem der Ansprüche 1 bis 11, **dadurch gekennzeichnet, daß** die Endsegmente (22, 23) und die Kupplungsstutzen (15) relativ zueinander koaxial angeordnet sind.

13. Vorrichtung nach einem der Ansprüche 1 bis 12, **dadurch gekennzeichnet, daß** das Filterelement (18) auswechselbar im Filtergehäuse (13) gehaltert ist.

14. Vorrichtung nach einem der Ansprüche 1 bis 13, **dadurch gekennzeichnet, daß** das Filtergehäuse (13) mit der Atemleitung (5) zusammensteckbar ist.

15. Vorrichtung nach einem der Ansprüche 1 bis 14, **dadurch gekennzeichnet, daß** die Kupplungselemente des Filtergehäuses (13) und der Atemleitung (5) Verschlüsse ausbilden.

16. Vorrichtung nach einem der Ansprüche 1 bis 15, **dadurch gekennzeichnet, daß** das Filtergehäuse (13) eine Arretierung (36) aufweist, die als ein federnd angeordneter Schwenkhebel ausgebildet ist.

## Claims

1. Apparatus for filtering respiratory air, which has a filter housing (13) comprising a housing lower part (16) and a housing upper part (17) and in which a filter element (18) is supported by the filter housing (13) and in which a line (21) for pressure transmission extends through the filter housing (13) and is equipped in the region of end segments (22, 23) in each case with a coupling (24, 25) for connection to a pressure measuring line (6), **characterized in that** one of the end segments (22, 23) is provided with a sealing element (28) and that the end segments (22, 23) of the line (21) for pressure transmission project into the coupling connection pieces (14, 15).

2. Apparatus according to claim 1, **characterized in that** the housing upper part (17) is provided with a coupling connection piece (15).

3. Apparatus according to claim 1 or 2, **characterized in that** the coupling connection piece (15) of the housing upper part (17) is designed as a coupling outer part.

4. Apparatus according to one of claims 1 to 3, **characterized in that** the housing lower part (16) comprises a coupling connection piece (14).

5. Apparatus according to one of claims 1 to 4, **characterized in that** the coupling connection piece (14) of the housing lower part (16) is designed as a coupling inner part.

6. Apparatus according to one of claims 1 to 5, **characterized in that** the sealing element (28) is mounted onto the end segment (22, 23).

7. Apparatus according to one of claims 1 to 6, **characterized in that** the sealing element (28) comprises a lead-in cone (30).

8. Apparatus according to claim 7, **characterized in that** the lead-in cone (30) is carried over in a funnel-shaped manner into a connecting recess (31).

9. Apparatus according to one of claims 1 to 8, **characterized in that** the sealing element (28) is formed from an elastomer.

10. Apparatus according to one of claims 1 to 9, **characterized in that** the line (21) for pressure transmission is of an at least two-part design.

11. Apparatus according to one of claims 1 to 10, **characterized in that** a line lower part (33) of the line (21) is intermateable with a line upper part (34).

12. Apparatus according to one of claims 1 to 11, **characterized in that** the end segments (22, 23) and the coupling connection pieces (15) are disposed coaxially relative to one another.

13. Apparatus according to one of claims 1 to 12, **characterized in that** the filter element (18) is mounted exchangeably in the filter housing (13).

14. Apparatus according to one of claims 1 to 13, **characterized in that** the filter housing (13) is intermateable with the respiratory line (5).

15. Apparatus according to one of claims 1 to 14, **characterized in that** the coupling elements of the filter housing (13) and of the respiratory line (5) form closures.

16. Apparatus according to one of claims 1 to 15, **characterized in that** the filter housing (13) has a locking device (36), which takes the form of a resiliently disposed pivoted lever.

## Revendications

1. Dispositif de filtration de l'air respiratoire, qui présente un boîtier de filtre (13) constitué d'une partie inférieure de boîtier (16) et d'une partie supérieure de boîtier (17), et dans lequel un élément de filtre (18) est maintenu dans le boîtier de filtre (13), un conduit (21) de transmission de la pression traversant le boîtier de filtre (13) et étant équipé, dans la zone de chacun de ses segments d'extrémité (22, 23) d'un accouplement respectif (24, 25) de raccordement à un conduit (6) de mesure de la pression, **caractérisé en ce que** l'un des segments d'extrémité (22, 23) est doté d'un élément d'étanchéité (28), et **en ce que** les segments d'extrémité (22, 23) du conduit (21) pénètrent dans les tubulures d'accouplement (14, 15) pour transmettre la pression.

2. Dispositif selon la revendication 1, **caractérisé en ce que** la partie supérieure de boîtier (17) est dotée d'une tubulure d'accouplement (15).

3. Dispositif selon la revendication 1 ou 2, **caractérisé en ce que** la tubulure d'accouplement (15) de la partie supérieure de boîtier (17) est configurée comme pièce extérieure d'accouplement.

4. Dispositif selon l'une des revendications 1 à 3, **caractérisé en ce que** la partie inférieure de boîtier (16) présente une tubulure d'accouplement (14).

5. Dispositif selon l'une des revendications 1 à 4, **caractérisé en ce que** la tubulure d'accouplement (14) de la partie inférieure de boîtier (16) est configurée comme pièce intérieure d'accouplement.

6. Dispositif selon l'une des revendications 1 à 5, **caractérisé en ce que** l'élément d'étanchéité (28) est enfiché sur le segment d'extrémité (22, 23).

7. Dispositif selon l'une des revendications 1 à 6, **caractérisé en ce que** l'élément d'étanchéité (28) présente un cône d'admission (30).

8. Dispositif selon la revendication 7, **caractérisé en ce que** le cône d'admission (30) se prolonge en entonnoir en une ouverture de liaison (31).

9. Dispositif selon l'une des revendications 1 à 8, **caractérisé en ce que** l'élément d'étanchéité (28) est réalisé en élastomère.

10. Dispositif selon l'une des revendications 1 à 9, **caractérisé en ce que** le conduit (21) de transmission de la pression est réalisé en au moins deux pièces.

11. Dispositif selon l'une des revendications 1 à 10, **caractérisé en ce qu'**une pièce inférieure (33) du conduit (21) peut être assemblée à une pièce supérieure (34) du conduit.

12. Dispositif selon l'une des revendications 1 à 11, **caractérisé en ce que** les segments d'extrémité (22, 23) et les tubulures d'accouplement (15) sont disposés coaxialement l'un par rapport à l'autre.

13. Dispositif selon l'une des revendications 1 à 12, **caractérisé en ce que** l'élément de filtre (18) est maintenu dans le boîtier de filtre (13) de manière à pouvoir être remplacé.

14. Dispositif selon l'une des revendications 1 à 13, **caractérisé en ce que** le boîtier de filtre (13) peut être raccordé au conduit respiratoire (5).

15. Dispositif selon l'une des revendications 1 à 14, **caractérisé en ce que** les éléments d'accouplement du boîtier de filtre (13) et du conduit respiratoire (5) forment des fermetures.

16. Dispositif selon l'une des revendications 1 à 15, **caractérisé en ce que** le boîtier de filtre (13) présente un arrêt (36) qui est configuré comme levier pivotant monté élastiquement.
